# EUROPEAN PATENT SPECIFICATION

(11) **EP 4 561 505 B1**
(45) Date of publication and mention of the grant of the patent: **16.09.2026**
(21) Application number: 23749047.9
(22) Date of filing: 28.07.2023
(51) Int. Cl.: A61F 2/88, A61F 2/04, A61M 27/00

(54) **UROLOGICAL STENT**
UROLOGISCHER STENT
STENT UROLOGIQUE

(30) Priority: 29.07.2022 EP 22306141
(43) Date of publication of application: 04.06.2025
(73) Proprietor: Bormo, 13090 Aix-en-Provence (FR)
(72) Inventor: MARCIANO, Pascal, 1030 Bussigny (CH)
(74) Representative: Icosa
(86) International application number: PCT/EP2023/070983
(87) International publication number: WO 2024/023290

(56) References cited:
- EP-B1- 1 110 561
- US-A1- 2003 040 803
- US-A1- 2004 260 384
- US-A1- 2009 210 045

## Description

### FIELD OF INVENTION

The present disclosure relates to a stent for insertion into the lumen of an anatomical vessel or duct, said stent comprising an expandable part capable of being in a compressed configuration during insertion, and capable of being in an expanded configuration after having been placed in the vessel or duct.

### BACKGROUND OF INVENTION

Stents are generally used for maintaining, or creating, a lumen in a body cavity. Stents are usually shaped as a tubular intraluminal prosthesis and are placed inside a vessel or duct of a human being or an animal. Stents may be used in a variety of body cavities, such as in the urinary canals, the blood vessels, etc., where occlusion of the body cavity may occur.

After placement, a stent needs to remain in place. Therefore, anchoring parts are disposed in order to ensure the strong anchoring of the stent inside the body cavity. Known stents may be anchored in a desired position inside the body cavity, after insertion, by expanding at least a part of the stent. However, stent migration is often observed: for instance, a stent for intraprostatic urethra being positioned between the external urethral sphincter and the bladder neck may migrate all the way into the bladder.

In addition, the mechanical flexibility of the stent should be balanced to support the anatomy of the body cavity where it is inserted and to follow the physical properties of the body cavity offering an overall flexibility to provide higher quality of life and in respect to physio-anatomical functions.

US patent application US2003/040803 discloses a urethra stent comprising a helical coil comprising a series of consecutive tuns made of a single wire. The diameter of the stent may be adjusted by twisting the helical coil, which recovers its diameter after releasing the twisting stress. The stent further comprises a soft webbing. Such stent requires a complex inserting kit, allowing to keep the stent twisted during placement. In addition, this stent is easily compressed under radial pressure - in a similar way as upon twisting. Last, the webbing may fold into pleats - especially when stent diameter is reduced - which limits flow of urine by limitation of the inner diameter of the stent and/or which creates stationary point of urine leading to infections.

US patent application US2009/210045 discloses urethral stents based on shape memory materials. However, these stents are prone to tissue incrustation between the turns of the wire, and cannot ensure a tight barrier between the interior of the stent and its exterior. In addition, the shape of said stent is ensured by a very compact structure, in which successive turns of wire are in contact, leading to an uncomfortable device, unable to bend and conform to the anatomy of the duct.

The stent herein disclosed provides a well-balanced performance with respect to retaining capacity, strong anchoring and overall flexibility, thus overcoming some or all disadvantages of the prior art.

### SUMMARY

The invention, as definined in the claims, relates to a stent comprising a polymer tube whose hollow part defines a longitudinal axis (Oz) and a single wire forming a helical coil. The stent is intended for insertion into the lumen of an urological vessel or duct. The helical coil comprises:
i. a series of consecutive turns embedded in a polymer tube; and
ii. at least one expandable part able to be either in a compressed configuration or in an expanded configuration, said expandable part being a shape-memory material.

In addition, the axis of the helical coil is substantially parallel to the longitudinal axis (Oz); and the polymer tube is radially rigid to prevent reduction of the diameter of the turns embedded in the polymer tube upon twisting of the stent around the longitudinal axis (Oz).

In other words, the wire is coiled in the wall of the polymer tube. And the polymer tube thus obtained is very stiff in the radial direction: it cannot collapse when pressure is applied radially - *i.e.,* when pressure is applied in a direction perpendicular to the longitudinal axis (Oz), or when the wire is twisted. But the polymer tube remains flexible in the axial direction: it can bend to conform to the shape of the lumen in which it is inserted. The stent is thus a composite device in which the polymer tube and the wire contribute synergically to obtain the required mechanical properties: radially rigid in order to resist compression - due to natural movement or due to a stenosis - and simultaneously flexible. Such flexibility is highly desirable in terms of comfort for the patient. For instance, in urological applications, the shape of urethra is changing depending on patient activity - sitting/standing, cycling, sexual relations - and stent 10 should adapt to these shape changes.

It is worth noting that the consecutive turns being embedded in the polymer tube, they cannot change of configuration from a compressed state to an expanded state. Therefore, the consecutive turns and the expandable part are different portions of the helical coil.

In an embodiment, the at least one expandable part is on the distal end of the stent or on the proximal end of the stent.

In an embodiment, the at least one expandable part is protruding in front of the distal end of the polymer tube or in front of the proximal end of the polymer tube.

In an embodiment, the at least one expandable part comprises a series of consecutive turns.

In an embodiment, the at least one expandable part comprises a series of consecutive turns coiled on the polymer tube in the compressed configuration.

In an embodiment, the inner surface of the polymer tube is cylindrical, preferably the inner surface of the polymer tube is cylindric and smooth. In an embodiment the outer surface of the polymer tube is partially or totally corrugated. In a preferred embodiment, the inner surface of the polymer tube is cylindric and smooth and the outer surface of the polymer tube is partially corrugated.

In an embodiment, the consecutive turns of helical coil embedded in the polymer tube have a constant pitch. Alternatively, the consecutive turns of helical coil embedded in the polymer tube have a first constant pitch in a first part of the polymer tube and have a second constant pitch in a second part of the polymer tube, the second constant pitch being different from the first constant pitch.

In an embodiment, the helical coil comprises at least one expandable part comprises a pair of turns in opposite directions and closed in a U-shape, so that the at least one expandable part in its expanded configuration defines a cylinder. In particular, the helical coil may comprise two U-shape expandable part, the second expandable part comprising a pair of turns in opposite directions and closed in a U-shape, so that the second expandable part in its expanded configuration defines a cylinder.

In an embodiment, the at least one expandable part comprises a series of consecutive turns in the compressed configuration and defines a conical shape in its expanded configuration.

In an embodiment, the at least one expandable part is coated with a polymer.

In an embodiment, the shape-memory material is selected from the group of polymers or metal alloys. Preferably the shape-memory material is a metal alloy of nickel and titanium.

This disclosure relates also to a kit comprising:
- A stent as disclosed hereabove;
- An introducer tool to introduce, deploy and manipulate the stent; and
- An irrigation channel through the introducer tool to flow liquid towards the stent.

### DEFINITIONS

In the present invention, the following terms have the following meanings:

**"Distal"** refers to the part of the stent that is located close to the orifice through which the stent is introduced, *i.e.,* close to the external meatus of genital system.

**"Expandable"** refers to a part of the stent whose dimension can increase from a compressed state to an expanded state. Expansion is triggered by an external stimulus, such as temperature change - thermally activated - or mechanical constraint - mechanically activated.

**"Flexibility"** refers to a general mechanical property, not exactly measured in this disclosure. Flexibility has to be understood as the property of the stent to be bendable or to follow the movements of the patient in respect to anatomical pathways and the physio-anatomical functions. A flexible stent should not be able to form a pleat, which is uncomfortable and limits flows of liquid.

**"French/Charrière",** abbreviated Fr, refers to the catheter scale for diameters: 1 mm equals 3 Fr. Therefore, the diameter of a round catheter in millimeters can be computed by dividing the French size by 3. Charrière is equivalent to French.

**"Helical coil"** refers to successive turns of a wire, said turns - or spires - being more or less regularly spaced, said turns being roughly oriented on a same axis, and said turns having roughly the same diameter. As a helical coil is deformable, its exact geometry is not well defined. In particular, a helical coil may be bent, stretched or compressed.

**"Pitch"** refers to the repetition length between two successive turns of a helical coil having regularly spaced turns.

**"Proximal"** refers to the part of the stent that is located distant from the orifice through which the stent is introduced, *i*.*e*., distant from the external meatus of genital system.

### DETAILED DESCRIPTION

This disclosure relates to a stent 10 for insertion into the lumen of an anatomical vessel or duct. The stent 10 comprises a polymer tube 30, whose hollow part defines a longitudinal axis (Oz); and a single wire 20 forming a helical coil. The helical coil comprises a series of consecutive turns embedded in. The axis of the helical coil is the direction around which the turns are coiled. The axis of the helical coil is substantially parallel to the longitudinal axis (Oz). The polymer tube 30 is intended to maintain and support the lumen of the tube cavity where the stent 10 is inserted: the polymer tube 30 is radially rigid to prevent reduction of the diameter of the turns embedded in the polymer tube 30 upon twisting of the stent around the longitudinal axis (Oz). Further, the polymer tube 30 remains flexible in the axial direction: it can bend to conform to the shape of the lumen in which it is inserted. In addition, the helical coil comprises an expandable part 22 able to be either in a compressed configuration or in an expanded configuration. This expandable part 22 is made of shape-memory material. Thus, the stent 10 may be inserted and placed while the expandable part 22 is compressed, in a low diameter shape. After placement, the shape-memory material may be triggered to adopt its expanded configuration. In the expanded zone, the stent 10 applies a radial load on the walls of the tube cavity and gets anchored. Last, the shape memory material may be triggered again to lose its shape for removal of the stent 10. For instance, expandable parts 22 may be designed to have a first shape - for introduction - at room temperature, a first trigger at high temperature - around 45°C or slightly above - to expand and keep the expanded state at body temperature; and a second trigger at low temperature - around 15°C or slightly below - where the expandable parts 22 lose their mechanical properties and become soft for removal. Indeed, softening the wire 20 eases stent removal because expandable parts 22 do not exert pressure anymore and wire 20 may be deformed in an elongated wire instead of a coil of large diameter.

In this disclosure, the consecutive turns are embedded in the polymer tube 30, they cannot change of configuration from a compressed state to an expanded state. Therefore, the consecutive turns and the expandable part 22 are different portions of the helical coil.

The stent 10 disclosed herein are especially suitable for urological applications, and may be placed in ureter, bladder neck or urethra.

In this disclosure, a single wire 20 is used. By single, it is meant that the whole coil is made of one wire, but said single wire 20 may be arranged in order to go back and forth along the longitudinal axis (Oz) of the polymer tube 30, for instance to form interweaved helixes. A single wire 20 is advantageous when stent 10 has to be removed. Indeed, the practitioner may pull on this wire 20 to move the stent 10. The polymer tube 30 will be pulled simultaneously, as the wire 20 is at least partially embedded in the polymer tube 30. In addition, the part of wire 20 embedded in the polymer tube 30 will not be elongated, and total length of the stent 10 for removal is reduced. Last, the risk of breakage during removal is strongly limited as the wire 20 is one single element.

The polymer tube 30 may be any suitable polymer known in the art. The polymer tube 30 may comprise additives to adapt surface properties: the polymer tube 30 may have hydrophilic and/or hydrophobic properties to improve an accelerated urine flow; to decrease incrustation and infection; and to provide strong anchoring forces. The polymer tube 30 may have tissues biocompatibility and Magnetic Resonance Imaging biocompatibility functions. The polymer tube 30 may be plasma treated. The polymer tube 30 may also contain drugs or actives that will be slowly released in the tissue where stent 10 is inserted, providing with therapeutic treatment.

Suitable polymers are for instance Thermoplastic Poly-Urethane (TPU) and Silicone.

The external diameter of the polymer tube 30 may be selected in a range from 8 Fr/CH to 26 Fr/CH. Especially suitable external diameters are 10.6, 24 Fr/CH.

The expandable part 22 may be located anywhere all along the polymer tube 30.

In an embodiment, the expandable part 22 is located on the end, distal end or proximal end, or elsewhere of the stent 10 to maintain a strong anchoring force that corresponds to the associated pathology in respect and taking into consideration the physio-anatomical constraints and properties. If the stent 10 comprises two expandable parts 22, they may be located on both ends - distal and proximal - or on one end and in the central part of the stent 10. For instance, expandable proximal part anchored at the bladder neck level, inside the bladder, is specially designed to enhance the flow of urine to urethra and to avoid any residual volume during urination. In the intra prostatic urethra area, the expandable distal part anchored above the external sphincter at the base of the prostate, is specially designed to prevent the risk of migration and the retrograde ejaculation. All the expandable parts 22 are especially designed to facilitate the insertion, expansion, and removal of the stent 10.

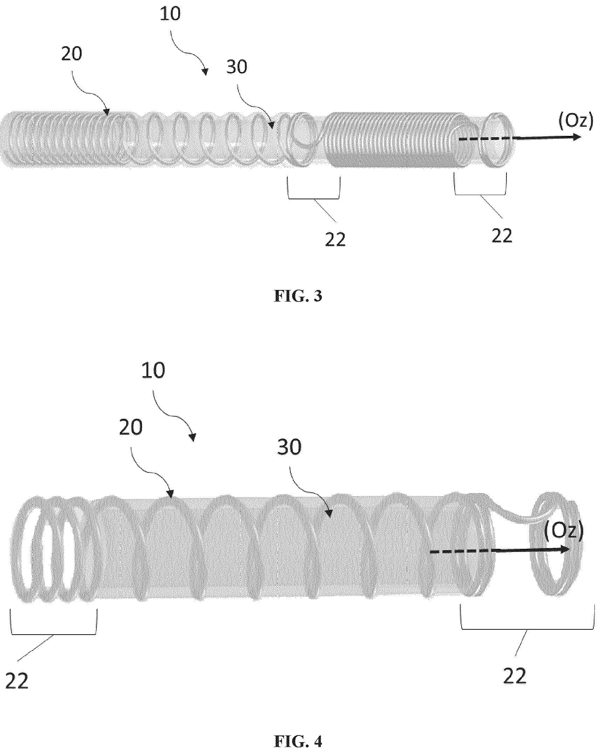

In an embodiment, the expandable part 22 is protruding in front of the distal end of the polymer tube 30 or in front of the proximal end of the polymer tube 30. This is advantageous when polymer tube 30 is intended to maintain and support the lumen of a cavity while the expandable part 22 is anchoring in a stronger and healthier area - with a different tissue resistance compared to the cavity where the polymer tube 30 is positioned. In particular, the expandable part 22 may comprise a series of consecutive turns. For instance, the helical coil may be partly embedded in the polymer tube 30 - retaining part - and partly protruding from the polymer tube 30 - expandable part 22. If the stent 10 comprises two expandable parts 22, they may be located on both ends - distal and proximal - or on one end and in the central part of the stent 10. Figures 4-5 are illustrations of a stent 10 with two expandable parts 22 protruding on both ends of the polymer tube 30. In urologic application, the configuration in which the expandable part 22 is protruding in front of the distal end of the polymer tube 30 is especially advantageous for drainage of urine: the consecutive turns of the distal end of the stent 10 help liquid drainage towards the polymer tube 30 and avoid any accumulation of urine which may cause incrustation and infection. In another urologic application, the configuration in which the expandable part 22 is protruding in front of the proximal end of the polymer tube 30 is especially advantageous to enable fluid flow between urethra and ducts arriving in urethra, for instance the ejaculatory duct. Indeed; in order to avoid retrograde ejaculation, the stent 10 shall not block sperm flow into urethra: the open structure of the expandable part 22 is thus appropriate to maintain the urethra open without closing ducts.

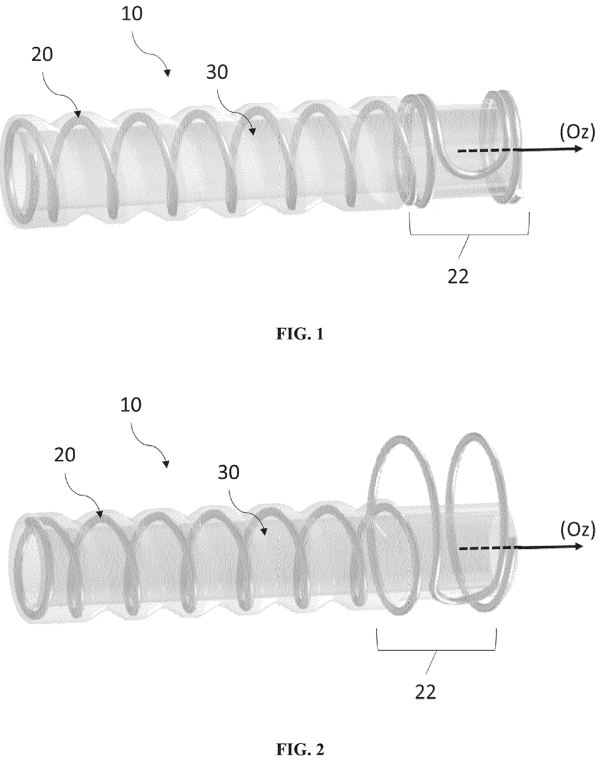

Alternatively, the expandable part 22 comprises a series of consecutive turns coiled on the polymer tube 30 in the compressed configuration to guaranty a uniform internal and external diameter of the insertion system and the stent 10. This will allow having an accurate optic visualization and easy insertion through the urinary pathway to avoid the need of dilatation prior to the insertion. The expandable part 22 may be located on the end, distal end or proximal end, or elsewhere of the stent 10. If the stent 10 comprises two expandable parts 22, they may be located on both ends - distal and proximal - or on one end and in the central part of the stent 10. Figures 1-2 are illustrations of a stent 10 with one expandable part 22 on one end of the polymer tube 30.

In this disclosure, the polymer tube 30 comprises at least one hollow part - sometimes referred to as a lumen in the stent domain - , through which liquid may flow or surgical instruments may be displaced. The hollow part defines a longitudinal axis (Oz). The helical coil is embedded in the walls of the polymer tube 30, so that the hollow part is in the inner part of the helical coil: the helical coil is substantially parallel to the longitudinal axis (Oz).

Obviously, the diameter of the turns of the helical coil embedded in the polymer tube 30 is greater than the inner diameter of the polymer tube 30.

The inner diameter of the polymer tube 30 may be selected in a range from 6 Fr/CH to 24 Fr/CH. Especially suitable inner diameters are 7, 18 Fr/CH.

The thickness of the polymer tube 30 may be selected in a range from 0.2 mm to 1.4 mm, preferably from 0.2 mm to 0.66 mm.

Obviously, the diameter of the turns of the helical coil embedded in the polymer tube 30 is defined by the diameter of the polymer tube 30. And the thickness of the polymer tube 30 is greater than the diameter of the wire 20 embedded in the polymer tube 30.

In an embodiment, the inner surface of the polymer tube 30 is cylindrical, preferably with a smooth surface. In another embodiment, the outside surface of the polymer tube 30 is corrugated, on a part of the polymer tube 30 or on the whole polymer tube 30.

The shape of surfaces has several advantages. A cylindrical smooth inner surface provides the highest flowrate for a given section; thus, flow of liquids is not impaired. In addition, cylindric smooth inner surface limits incrustation - and then avoids accumulation of minerals salts and crystalline deposits originating from the urine for instance - potentially leading to obstruction that consequently leads to infection due to urine stagnation - inside the stent 10. A corrugated outer surface provides with a better contact with the walls of the cavity to be retained and avoids movements of the stent 10. Such a corrugated outer surface therefore limits migration of the stent 10 and contributes to a strong anchoring.

In a preferred embodiment, the inner surface of the polymer tube 30 is hydrophilic - to improve an accelerated urine flow and to decrease incrustation for instance - and the outside surface is hydrophobic - to avoid urine flow between the stent 10 and the tissues and to provide strong anchoring forces for instance.

In a preferred embodiment, the corrugated outer surface of the polymer tube 30 is defined in correspondence with the turns of the helical coil embedded in the polymer tube 30: a peak in the outer surface appears where the wire 20 is located, whereas a valley in the outer surface appears between two consecutive turns of the wire 20.

In this disclosure, the flexibility of the stent 10 - bending ability along the longitudinal axis of the polymer tube 30 - results from the intrinsic mechanical properties of the polymer tube 30 - material, thickness... - and wire 20 - material, diameter... - as well as the geometry of the wire 20 embedded in the polymer tube 30, forming a composite device. Obviously, if consecutive turns of the helical coil embedded in the polymer tube 30 are in contact, the helical coil will not be able to bend easily, leading to low flexibility of the stent 10. To the contrary, if consecutive turns are well separated by a flexible polymer, bending will be governed by polymer properties, and the stent 10 may be very flexible. The geometry of the wire 20, embedded in the polymer tube 30, allows the design of the stent 10 to conform to the associated pathology, with variable degree of flexibility, in respect with the physio-anatomical properties like peristaltic movements of the ureter, as well as the associated pathology, like but not limited to stenosis, extrinsic pressure from adjacent organs... The flexibility of the stent 10 may include the deformation - essentially bending or peristaltic undulations - of the polymer tube 30. However, the polymer tube 30 is radially rigid. In particular, the stent 10 may be used to oppose stenosis: if a duct is closed - or almost closed - the stent 10 will create an open channel through the polymer tube 30, and the mechanical properties of the polymer tube 30 reinforced with the wire 20 ensure that the channel remains open. This is especially relevant for the case of urine drainage. During urination, the flow of urine is governed by a balance between bladder pressure and urethra opening. In case of stenosis, a residual volume of urine remains in the bladder, potentially leading to infections or complications like kidney stones. Keeping open the channel of the stent 10 avoids any retention of urine in the bladder. A polymer tube 30 of inner diameter 18 Fr/CH is especially adapted for this case.

In an embodiment, the consecutive turns of helical coil embedded in the polymer tube 30 have a constant pitch. In this embodiment, the flexibility of the stent 10 will be uniform. This embodiment is suitable when a retaining structure is expected to impose a uniform load on the walls of the tube cavity where it is inserted, without point of low pressure, that could lead to discomfort for the patient or fatigue crack of the stent 10.

In another embodiment, the consecutive turns of helical coil embedded in the polymer tube 30 have a first constant pitch in a first part of the polymer tube 30 and have a second constant pitch in a second part of the polymer tube 30, the second constant pitch being different from the first constant pitch. This embodiment defines two domains with different flexibility along the stent 10. This embodiment is especially advantageous when the stent 10 has to support the walls of a cavity on one part and follow movements of the patient on another part in respect with the physio-anatomical properties.

More generally, the helical coil may define more than two parts, each part having a constant pitch. The pitch of the helical coil may be also variable, according to a predefined design, thereby providing "on demand" flexibility along the stent 10.

In an embodiment, the expandable part 22 comprises a pair of turns in opposite directions and closed in a U-shape. In its expanded configuration, the expandable part 22 thus adopts a cylindrical shape, defined by the two circles corresponding to the two expanded turns, thereafter referred to as U-shape geometry. Such a cylindrical shape is especially advantageous to impose a load on a large surface of contact with the walls of a cavity, avoiding migration of the wire 20 and providing with better anchoring over a large area.

The U-shape expandable part 22 also allows in some cases to have liquid flow outside the polymer tube 30. Indeed, anchoring is ensured by simple wires 20 which do not plug a possible flow path between the outside surface of the stent 10 and the tissues. This is particularly relevant for a prostatic stent 10 which shall not induce retrograde ejaculation.

For U-shape geometry, the external diameter of the expandable part 22 in its compressed configuration may be selected in a range from 8 Fr/CH to 26 Fr/CH. Especially suitable external diameters are 10.6, 24 Fr/CH. After expansion, the diameter of the expandable part 22 may be selected in a range from 18 Fr/CH to 44 Fr/CH. Typically, external diameter in expanded configuration is greater than the external diameter in compressed configuration by a factor ranging from 1.5 to 2. Preferred couples for diameters in compressed configuration and expanded configuration are 10.6/21 Fr/CH 24/42 Fr/CH.

In an embodiment, the cylinder defined by the two expanded turns and the polymer tube 30 are coaxial.

Alternatively, the cylinder defined by the two expanded turns may be not coaxial with the polymer tube 30. In this specific configuration, the stent 10 may be placed inside the cavity while keeping a lumen between the stent 10 and the walls of the cavity. If the polymer tube 30 is not a tube - the case of a drain for instance - this configuration allows liquid flow on the outer surface of the polymer tube 30. If the polymer tube 30 is a tube, this configuration allows two distinct flow paths: inside the tube and outside the tube, which is especially suitable if a stent 10 is placed where two vessels or ducts join.

In an embodiment, the helical coil comprises two or more expandable parts 22, each expandable part 22 comprising a pair of turns in opposite directions and closed in a U-shape as disclosed hereabove. In this embodiment, the stent 10 is anchored to the cavity on two or more cylindric zones. Thus, each anchoring point impose a lower load on the walls of a cavity, but all anchoring point together provide with a strong anchoring. In addition, the two U-shape expandable parts 22 define a precise placement of the stent 10 and avoids any movement - or migration - of the stent 10. It allows also to keep the exact structure of the turns of helical coil: the more or less flexible parts of the stent 10 are thus precisely located. Each U-shaped expandable part 22 may be coaxial with the polymer tube 30, or not.

In an embodiment, the expandable part 22 comprises a series of consecutive turns in the compressed configuration and defines a conical shape in its expanded configuration, thereafter referred to as conical geometry. Such conical shape is especially advantageous when a duct or vessel is connected to a larger cavity. The conical shape may expand in the large cavity, thereby avoiding migration of the stent 10 into the vessel or duct.

For conical geometry, the external diameter of the expandable part 22 in its compressed configuration may be selected in a range from 8 Fr/CH to 24 Fr/CH. Especially suitable external diameters are 10.6, 24 Fr/CH. After expansion, the external diameter of the largest turn of the expandable part 22 may be selected in a range from 18 Fr/CH to 44 Fr/CH. Typically, diameter of the largest turn in expanded configuration is greater than the diameter in compressed configuration by a factor ranging from 1.5 to 2. Preferred couples for diameters in compressed configuration and the largest turn in expanded configuration are 10.6/21 Fr/CH 24/42 Fr/CH.

More generally, the stent 10 of the disclosure may have several expandable parts 22, selected from the group of cylindrical, U-shape or conical geometries.

For examples, a stent 10 according to this disclosure may comprise
- a U-shape geometry on the distal end and another U-shape geometry on the proximal end or in the middle of the stent;
- a U-shape geometry on the distal end and a conical geometry on the proximal end;
- a U-shape geometry on the proximal end and a conical geometry on the distal end; or
- a conical geometry on the distal end and another conical geometry on the proximal end.

In all these examples, cylindrical geometries, U-shape geometries and conical geometries may be protruding from the polymer tube 30 or may be coiled on the polymer tube 30.

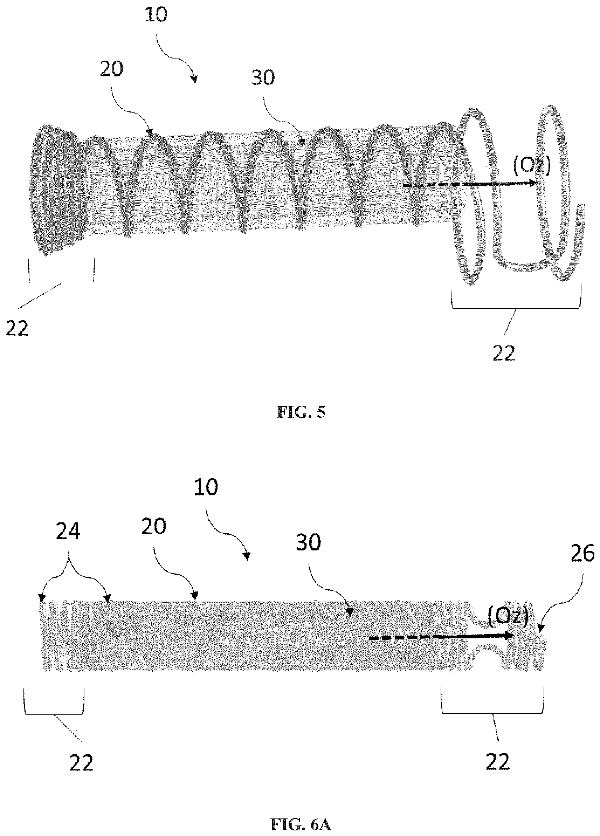

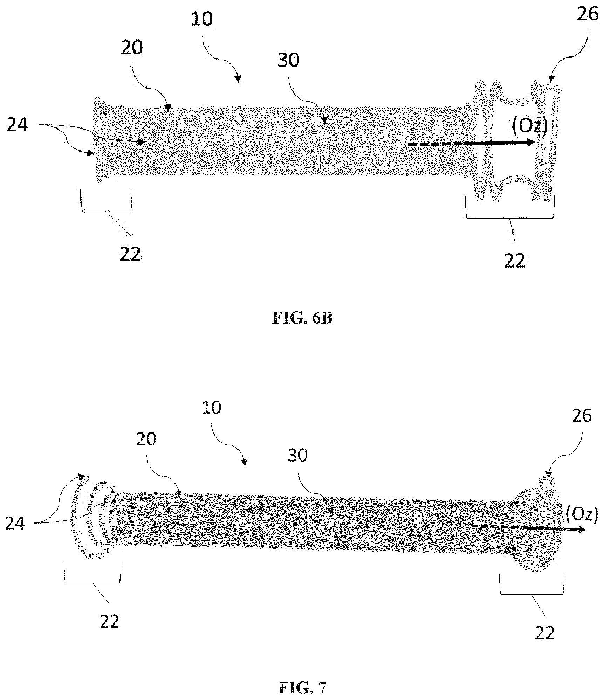

In an embodiment, the helical coil is formed from a single wire 20, but comprises a loop 26, preferably on the distal end of the stent 10. In this embodiment, the wire 20 is bent to form a loop 26 at one end of the stent 10. Such a loop 26 provides several advantages. First, the free end 24 of the wire 20 is not sharp cut, which may avoid any risk for tissue injuries of the wire 20 in the tissues or sensitive and uncomfortable points. Second, the loop 26 is a practical point to grasp the stent 10 for an easy removal: indeed, during operation, a simple hook may be used to enter the loop 26 and allow easy traction on the wire 20 for easy removal of the stent 10. This feature is clearly visible on figure 7, distal end. Third, the wire 20 being bent, its apparent length is shorter. In other words, after elongation of the helical coil, the length of the wire 20 with a loop 26 - from the loop 26 to the free end 24 of the single wire 20 - may be half the length of the same wire 20 without a loop 26. During removal of the stent, a shorter length is desirable for comfort of the patient and ease of operation. This feature is clearly visible on figures 6 and 10-12: the U-shape distal end is actually a loop. The helical coil in the polymer tube 30 is the interweaving of two helixes.

When the end of the stent 10 is a U-shape geometry with a loop 26, the expanded configuration may comprise two pairs of turns in opposite directions and closed in a U-shape - visible on figures 6 and 10-12 - providing a better anchoring. Indeed, both U-shape sections are facing each other, and form two reinforcements for the same section of the stent 10. Such structure cannot be pinched, thus provides with a more stable anchoring effect and placement. Last, the height of such an expandable part 22 - measured along the longitudinal axis (Oz) - does not change significantly during expansion: after placement of the stent 10 in the compressed configuration, the expandable part 22 is expanded and stays in the exact position of placement. The accuracy of placement is thus improved. An expandable part 22 of expanded diameter 42 Fr/CH is especially adapted for this case.

In an embodiment, the helical coil is formed from a single wire 20, but comprises two loops 26: one loop 26 on the distal end of the stent 10 and one loop 26 on the proximal end of the stent 10. A loop 26 located on the proximal end of the stent 10 may help the handling and precise placement of the stent 10.

Conical geometries may have additional characteristics.

In an embodiment illustrated on figure 6, distal end, the conical geometry of the expanded part forms a duct with continuous walls. In this geometry, the expandable part 22 expands in such a way that spires contract along the axis of the helical coil and get in contact, without space between spires. Such a geometry provides a sealing effect and prevent liquid flow through the helical coil and tissue proliferation. This effect is further improved when the expandable part 22 of the stent 10 is coated with a polymer, the latter having eventually hydrophobic/hydrophilic properties and/or compressible properties, thus acting as a seal. This feature is especially interesting on the proximal end of the stent, to avoid liquid dispersion around the stent, eventually leading to liquid trapping, which shall be prevented to avoid potential infection around the stent.

Alternatively, the conical geometry of the expanded part forms a duct with well-separated spires, as illustrated on figure 7, proximal end. In this geometry, the expanded part behaves like a drain to help liquid flow in direction of the polymer tube. This feature is especially interesting on the proximal end of the stent, to avoid any liquid accumulation. For instance, if the conical expanded part is placed inside the bladder, just above the bladder neck, drainage of urine will be complete. Preferably, the conical expanded part is protruding in front of the distal end of the polymer tube 30.

In an embodiment, the expandable part 22 is coated with a polymer. In particular, the expandable part 22 may be coated with the same polymer as the one used for the polymer tube 30, eventually comprising additives. Preferably, the coating thickness is lower than 300 µm, so that mechanical properties of the coating are negligible and do not impair expansion or compression of the expandable part 22.

Regarding shape-memory material, polymers and metal alloys are suitable. Polymeric shape-memory materials may be selected in the group of polyurethanes, polynorbornene or cross-linked polyethyleneoxide (PEO)-polyethyleneterephthalate (PET) block copolymers. Metal alloy shape-memory materials may be selected in the group of copper-aluminium-nickel and nickel-titanium. Metal alloys of nickel and titanium - known under the generic Nitinol term - are especially adapted.

Preferred shape-memory materials are selected among thermally activated shape-memory materials. In particular, use in human body is optimum when shape-memory material is shaped at room temperature and keeps its stressed structure up to usual internal human temperature - up to 40°C. After placement of the stent 10, the stent could be expanded thermally using a heated liquid may be circulated around the stent 10, with temperature above 40°C but low enough to avoid any lesion, thereby inducing expansion of the expandable part 22. The preferred transition temperature for shape-memory material is in the range from 45°C to 65°C.

Other suitable shape-memory materials are selected among mechanically activated shape-memory materials. In this case, the stent 10 is placed in the human body, then mechanically excited - by a force, a torsion or vibrations for instance - thereby releasing internal stresses and triggering shape change.

In an embodiment, expansion is not reversible: after expansion with heated liquid or mechanical constraint, stent 10 keeps its expanded configuration.

The stents 10 disclosed hereabove can be easily removed from the body. Upon cooling, typically below 15°C, the shape-memory material loses its rigidity, become elastic and may be deformed with very small force. Thus, the practitioner may remove a stent 10 according to the invention by cooling the stent, then grasping the wire 20 - eventually by a loop 26 - and pulling. The wire 20 will deform and lose its helical structure. Besides, as part of the wire 20 is embedded in the polymer tube 30, pulling on the wire 20 also pull the polymer tube 30, which is not reinforced mechanically by the wire, thus more easily deformable, and more easily extracted. This effect is especially desirable in case of persistent stenosis, because the stent 10 is under pressure from the body and traction on the stent 10 may hurt the patient. After softening of the expandable parts 22 and the wire 20, removal of stent 10 gets safer and easier.

In order to expedite removal of the stent, the polymer tube 30 may be segmented in successive parts. In the compressed and expanded configurations, strains in the wire 20 keep the successive parts in contact: the stent 10 behaves as if only one polymer tube 30 were used - or one polymer tube. However, after cooling of the stent 10 and relaxation of the strains in the wire, the successive parts acquire some relative mobility and may be removed more comfortably.

The disclosure also relates to a method of fabrication of a stent 10 as disclosed hereabove. This method comprises the following steps:
i. Shaping a shape memory wire 20 into a helical coil;
ii. Forming a polymer tube 30 with a helical groove on the outside surface corresponding to the helical coil;
iii. Inserting the helical coil into the helical groove of the polymer tube 30; and
iv. Depositing polymer on the helical coil and the polymer tube 30 so as to embed the helical coil into the polymer tube 30;
wherein the helical coil comprises at least one expandable part 22 able to be either in a compressed configuration or in an expanded configuration.

The step of forming the polymer tube 30 may be molding - with a mold having grooves - cutting a groove in a polymer tube 30, embossing a polymer tube 30 or additive manufacturing. Alternatively, the polymer tube 30 may be molded with the wire already placed in the mold.

The step of depositing polymer on the helical coil and the polymer tube 30 may be over molding, coating - such as dip coating or spray coating - or additive manufacturing.

In an embodiment, the method of fabrication further comprises a functionalization step, which may be selected from: applying a hydrophilic treatment to the inner surface of the stent; applying a hydrophobic treatment to the outer surface of the stent; or applying a plasma treatment to the polymer.

In an embodiment, the method of fabrication further comprises a step of incorporation of an active to be delivered slowly. For instance, such an active may be embedded within the polymer tube 30 and released by diffusion through the polymer material. Alternatively, such an active may be deposited on the polymer tube 30 in a polymeric matrix - in the form of a film or coating - leading to controlled release.

The disclosure also relates to a kit comprising:
- A stent 10 as disclosed hereabove;
- An introducer tool to introduce, deploy and manipulate the stent; and
- An irrigation channel through the introducer tool to flow liquid towards the stent 10.

The introducer tool can include a distal end adapted to selectively receive or secure the stent 10 therein, a handle having one or more actuation portions (e.g., trigger or actuators), and a tubing or shaft portion. The tubing or shaft portion can be generally flexible and in operable communication with the handle and the stent 10 to facilitate control and deployment of the stent 10 within the vessels or ducts of the patient.

### BRIEF DESCRIPTION OF THE DRAWINGS

**Figure 1** is a schematic showing a stent 10 according to example 1, in the compressed configuration.
**Figure 2** is a schematic showing a stent 10 according to example 1, in the expanded configuration.
**Figure 3** is a schematic showing a stent 10 according to example 2, in the compressed configuration.
**Figure 4** is a schematic showing a stent 10 according to example 3, in the compressed configuration.
**Figure 5** is a schematic showing a stent 10 according to example 3, in the expanded configuration.
**Figure 6** is a schematic showing a stent 10 according to a variant of example 3 designed with a loop 26, in the compressed configuration (Figure 6A) and in the expanded configuration (Figure 6B).
**Figure 7** is a schematic showing a stent 10 according to example 4, in the expanded configuration.
**Figure 8** is a schematic showing a stent 10 according to example 5, in the expanded configuration.
**Figure 9** is a schematic showing a stent 10 according to example 6, in the expanded configuration.
**Figure 10** is a schematic showing a stent 10 according to example 7, in the expanded configuration.
**Figure 11** is a schematic showing a stent 10 according to example 8, in the expanded configuration.
**Figure 12** is a schematic showing a stent 10 according to example 9, in the expanded configuration.

On all figures, stents 10 are presented with the proximal side on the left and the distal side on the right. Longitudinal axis (Oz) is represented pointing towards the distal side.

### EXAMPLES

The present invention is further illustrated by the following examples.

All examples are prepared with Thermoplastic Poly-Urethane (TPU) polymer for the polymer tube 30 and optionally the polymer coating and with 0.66 mm diameter nitinol as wire 20 for the helical coil.

### Example 1

As shown in Figure 1 in its compressed configuration, the stent 10 is 40 mm shaft length (excluding the expandable parts 22) and comprises a polymer tube 30- inner diameter 18Fr/CH, outer diameter 24Fr/CH, outer surface corrugated with undulation amplitude of 0.66 mm - and a nitinol wire 20. Seven consecutive turns of the helical coil are embedded in the polymer tube 30, with a constant pitch of 5 mm. In addition, the expandable part 22 is a U-shape geometry coiled on the polymer tube 30 - distal end - with both turns separated by 4 mm and an outer diameter in compressed configuration of 24 FR/CH. Here, the expandable part 22 is not coated.

Upon thermal activation with physiological serum heated at 50°C, the expandable part 22 expands and reaches an outer diameter of 42 Fr/CH, as shown in Figure 2. It can be observed that polymer tube 30 and expanded part in its expanded configuration are not coaxial.

Similar stents 10 of length 40 mm, 50 mm 60 mm and 70 mm have been prepared by proper selection of the number of consecutive turns of the helical coil embedded in the polymer tube 30.

This stent 10 is intended to be used in case of Benign Prostatic Hyperplasia.

### Example 2

As shown in Figure 3 in its compressed configuration, the stent 10 is 60 mm long and comprises a polymer tube 30- inner diameter 18Fr/CH, outer diameter 24Fr/CH - and a nitinol wire 20. The stent 10 comprises three different domains.

A low-flexibility domain is located between two expandable parts 22. The low-flexibility part is comprised of twenty consecutive turns in contact, embedded in the polymer tube 30. The expandable parts 22 are identical, namely coiled on the polymer tube 30 with a U-shape geometry coiled on the polymer tube 30 - distal end and in the middle - with both turns separated by 4 mm and an outer diameter in compressed configuration of 24 FR/CH. Here, the expandable parts 22 are not coated.

On the central domain, a very flexible domain, adjacent to the expandable part 22 located in the middle of the stent, is comprised of seven consecutive turns of the helical coil embedded in the polymer tube, with a constant pitch of 5 mm.

On the proximal side, a semi-flexible domain, adjacent to the flexible domain, is comprised of fourteen consecutive turns of the helical coil embedded in the polymer tube 30, with a constant pitch of 2 mm.

Upon thermal activation with physiological serum heated at 50°C, the expandable parts 22 expand and reaches an outer diameter of 42 Fr/CH.

This stent 10 shows different flexibility along its length.

The central structure of this stent, namely flexible domain and expandable part 22 located in the middle of the stent, correspond to the stent 10 of example 1.

### Example 3

As shown in Figure 4 in its compressed configuration, the stent 10 is 40 mm shaft length (excluding the expandable parts 22) and comprises a polymer tube 30- inner diameter 18Fr/CH, outer diameter 24Fr/CH - and a nitinol wire of 0.66 mm. 7 consecutive turns of the helical coil are embedded in the polymer tube, with a constant pitch of 5 mm. In addition, two expandable parts 22 are prepared. On the distal end, a U-shape geometry is protruding from the polymer tube 30 with both turns separated by 4 mm and an outer diameter in compressed configuration of 24 FR/CH. On the proximal end, a conical geometry has 3 consecutive turns protruding from polymer tube 30, with a pitch of 1.32 mm and an outer diameter in compressed configuration of 24 FR/CH

Here, the distal U-shape geometry is not coated, whereas the proximal conical geometry is polymer coated.

Upon thermal activation with physiological serum heated at 50°C, the expandable parts 22 expand and reach an outer diameter of 42 Fr/CH for the distal U-shaped geometry and a conical structure for the proximal conical geometry, as shown in Figure 5. It can be observed that polymer tube 30 and U-shape geometry expanded part in its expanded configuration are not coaxial.

In a variant shown on figure 6 (in compressed configuration 6A and in expanded configuration 6B), the distal end comprises a loop 26 and two U-shape geometries, in a coaxial direction with the polymer tube. Here, two helixes are interweaved in the polymer tube 30, yielding height spires along the tube. Stent 10 of figure 5 shows also height spires along the tube: the flexibility of both stents 10 is comparable. However, during removal, the total length of wire 20 of stent 10 of figure 6 - from loop 26 to free ends 24 of the wire 20 - is almost half of the total length of the wire 20 of stent 10 of figure 5.

Similar stents 10 of length 40 mm, 50 mm 60 mm and 70 mm have been prepared by proper selection of the number of consecutive turns of the helical coil embedded in the polymer tube 30.

### Example 4

As shown in Figure 7 in its expanded configuration, the stent 10 is 70 mm shaft length (excluding the expandable parts 22) and comprises a polymer tube 30- inner diameter 18Fr/CH, outer diameter 24Fr/CH - and a nitinol wire. The helical coil embedded in the polymer tube 30 has a variable spire spacing along the axis of the tube: flexibility of the stent 10 is higher in the middle as compared to the ends. The stent 10 comprises two expandable parts 22: a conical geometry with a loop 26 leading to a sealed structure and a conical geometry leading to a draining structure.

This stent 10 is intended to be placed at the bladder base, with the draining expanded part in the bladder - proximal end - and the sealed expanded part in urethra - distal end. The loop 26 on distal end allows for easy grasping of the stent 10 for removal. This stent 10 is particularly suitable for the treatment of Detrusor Sphincter Dyssynergia (DSD).

### Example 5

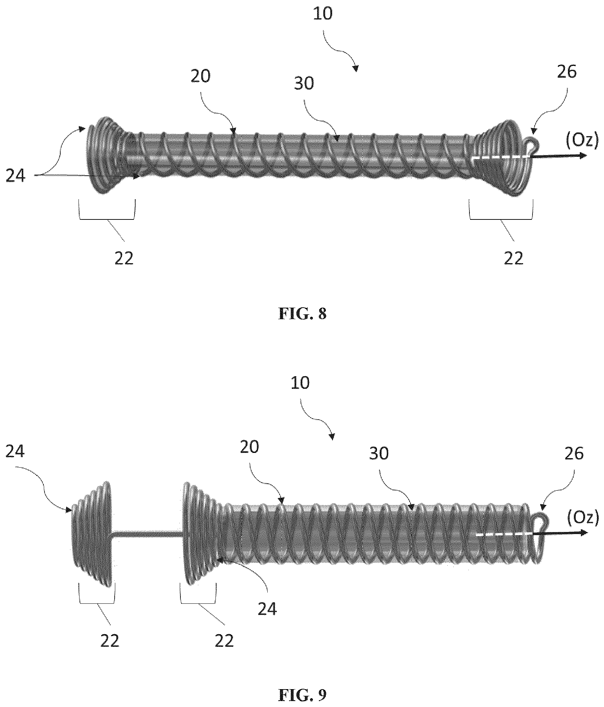

As shown in Figure 8 in its expanded configuration, the stent 10 of example 4 is adapted with a 0.40 mm diameter nitinol as wire 20 with a constant spire spacing and a polymer tube 30 - inner diameter 7 Fr/CH, outer diameter 10.6 Fr/CH - flexible enough to adapt to the anatomy and peristaltic movements of ureter, with a length of 30 mm. The stent 10 comprises two expandable parts 22: a conical geometry with a loop 26 leading to a sealed structure and a conical geometry leading to a sealed structure. This stent 10 is particularly suitable for the treatment of ureter stenosis.

### Example 6

As shown in Figure 9 in its expanded configuration, the stent 10 is 70 mm shaft length (excluding the expandable parts 22) and comprises a polymer tube 30- inner diameter 18Fr/CH, outer diameter 24Fr/CH - and a nitinol wire. The stent 10 comprises two expandable parts 22 of conical geometry facing each other. In addition, the distal end of the stent 10 has a loop 26.

This stent 10 is intended to be placed at the urinary sphincter, with the expandable conical part located from either side of the sphincter, thereby ensuring a very strong anchoring. This stent 10 is particularly suitable for the treatment of urethra stenosis.

### Example 7

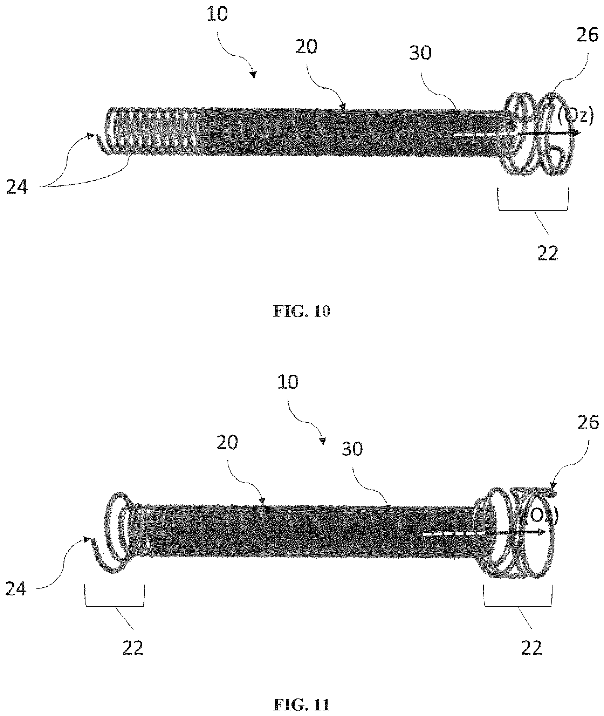

As shown in Figure 10 in its expanded configuration, the stent 10 is 70 mm shaft length (excluding the expandable parts 22) and comprises a polymer tube 30- inner diameter 18Fr/CH, outer diameter 24Fr/CH - and a nitinol wire. The stent 10 comprises one expandable part 22 of U-shape geometry on the distal end. In addition, the distal end of the stent 10 has a loop 26. On the proximal side, the wire 20 is protruding from the polymer tube 30 and the spire spacing is shorter than in the tube, to adapt flexibility of the stent 10 to the anatomy. This stent 10 is particularly suitable for the treatment of Benign Prostatic Hyperplasia with an extension in the median lobe.

### Example 8

As shown in Figure 11 in its expanded configuration, the stent 10 is 70 mm shaft length (excluding the expandable parts 22) and comprises a polymer tube 30- inner diameter 18Fr/CH, outer diameter 24Fr/CH - and a nitinol wire. The stent 10 comprises one expandable part 22 of U-shape geometry with a loop 26 on the distal end and a conical geometry leading to a draining structure on the proximal end. This stent 10 is particularly suitable for the treatment of Benign Prostatic Hyperplasia associated with stenosis of bladder neck. Thus, the spire spacing is shorter on the proximal side intended to be placed in the bladder neck.

### Example 9

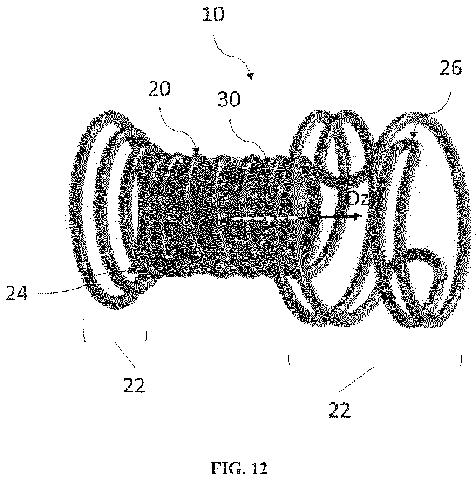

As shown in Figure 12 in its expanded configuration, the stent 10 of example 8 is adapted with a shorter polymer tube 30 to have a 20 mm shaft length (excluding the expandable parts 22). This stent 10 is particularly suitable for the treatment of stenosis of bladder neck.

### Numerical references:

10 : Stent / 20: Wire / 22: Expandable part / 24: free end of wire / 26: Loop / 30: Polymer tube.

## Claims

1. A stent (10) for insertion into the lumen of a urological vessel or duct comprising:
• a polymer tube (30) whose hollow part defines a longitudinal axis (Oz)
• a single wire (20) forming a helical coil comprising
i. a series of consecutive turns embedded in the polymer tube (30); and
ii. at least one expandable part (22) able to be either in a compressed configuration or in an expanded configuration, said expandable part being a shape-memory material;
wherein the axis of the helical coil is substantially parallel to the longitudinal axis (Oz); and
wherein the polymer tube (30) is radially rigid to prevent reduction of the diameter of the turns embedded in the polymer tube (30) upon twisting of the stent (10) around the longitudinal axis (Oz).

2. The stent (10) according to claim **1,** wherein said at least one expandable part (22) is on the distal end of the stent (10) or on the proximal end of the stent (10).

3. The stent (10) according to claim **1** or **2,** wherein the at least one expandable part (22) is protruding in front of the distal end of the polymer tube (30) or in front of the proximal end of the polymer tube (30).

4. The stent (10) according to claim **3,** wherein the at least one expandable part (22) comprises a series of consecutive turns.

5. The stent (10) according to claim **1** or **2,** wherein the at least one expandable part (22) comprises a series of consecutive turns coiled on the polymer tube (30) in the compressed configuration.

6. The stent (10) according to any one of claims **1** to **5,** wherein
• the inner surface of the polymer tube (30) is cylindric, preferably the inner surface of the polymer tube (30) is cylindric and smooth; and/or
• the outer surface of the polymer tube (30) is partially or totally corrugated.

7. The stent (10) according to any one of claims **1** to **6,** wherein the consecutive turns of helical coil embedded in the polymer tube (30) have a constant pitch.

8. The stent (10) according to any one of claims **1** to **6,** wherein the consecutive turns of helical coil embedded in the polymer tube (30) have a first constant pitch in a first part of the polymer tube (30) and have a second constant pitch in a second part of the polymer tube (30), the second constant pitch being different from the first constant pitch.

9. The stent (10) according to any one of claims **1** to **8,** wherein the at least one expandable part (22) comprises a pair of turns in opposite directions and closed in a U-shape, so that the at least one expandable part (22) in its expanded configuration defines a cylinder.

10. The stent (10) according to claim **9,** further comprising a second expandable part (22) comprising a pair of turns in opposite directions and closed in a U-shape, so that the second expandable part (22) in its expanded configuration defines a cylinder.

11. The stent (10) according to any one of claims **1** to **8,** wherein the at least one expandable part (22) comprises a series of consecutive turns in the compressed configuration and defines a conical shape in its expanded configuration.

12. The stent (10) according to any one of claims **1** to **11,** wherein the at least one expandable part (22) is coated with a polymer.

13. The stent (10) according to any one of claims **1** to **12,** wherein the shape-memory material is selected from the group of polymers or metal alloys, preferably the shape-memory material is a metal alloy of nickel and titanium.

14. A kit comprising:
• A stent (10) according to any one of claims **1** to **13;**
• An introducer tool to introduce, deploy and manipulate the stent (10); and
• An irrigation channel through the introducer tool to flow liquid towards the stent (10).

## Patentansprüche

1. Stent (10) zum Einführen in das Lumen eines urologischen Gefäßes oder Ganges, umfassend:
• ein Polymerrohr (30), dessen Hohlraum eine Längsachse (Oz) definiert;
• einen einzelnen Draht (20), der eine spiralförmige Spule bildet, umfassend:
i. eine Reihe aufeinanderfolgender Windungen, die in das Polymerrohr (30) eingebettet sind; und
ii. mindestens einen expandierbaren Teil (22), der entweder in einer komprimierten Konfiguration oder in einer expandierten Konfiguration vorliegen kann, wobei der expandierbare Teil aus einem Formgedächtnismaterial besteht;
wobei die Achse der spiralförmigen Spule im Wesentlichen parallel zur Längsachse (Oz) verläuft; und
wobei das Polymerrohr (30) radial starr ist, um eine Verringerung des Durchmessers der in das Polymerrohr (30) eingebetteten Windungen beim Verdrehen des Stents (10) um die Längsachse (Oz) zu verhindern.

2. Stent (10) nach Anspruch 1, wobei sich der mindestens eine expandierbare Teil (22) am distalen Ende des Stents (10) oder am proximalen Ende des Stents (10) befindet.

3. Stent (10) nach Anspruch 1 oder 2, wobei der mindestens eine expandierbare Teil (22) vor dem distalen Ende des Polymerrohrs (30) oder vor dem proximalen Ende des Polymerrohrs (30) hervorsteht.

4. Stent (10) nach Anspruch 3, wobei der mindestens eine expandierbare Teil (22) eine Reihe aufeinanderfolgender Windungen umfasst.

5. Stent (10) nach Anspruch 1 oder 2, wobei der mindestens eine expandierbare Teil (22) eine Reihe aufeinanderfolgender Windungen umfasst, die in der komprimierten Konfiguration auf das Polymerrohr (30) aufgewickelt sind.

6. Stent (10) nach einem der Ansprüche 1 bis 5, wobei die Innenfläche des Polymerrohrs (30) zylindrisch ist, vorzugsweise die Innenfläche des Polymerrohrs (30) zylindrisch und glatt ist; und/oder die Außenfläche des Polymerrohrs (30) teilweise oder vollständig gewellt ist.

7. Stent (10) nach einem der Ansprüche 1 bis 6, wobei die aufeinanderfolgenden Windungen der in das Polymerrohr (30) eingebetteten spiralförmigen Spule eine konstante Steigung aufweisen.

8. Stent (10) nach einem der Ansprüche 1 bis 6, wobei die aufeinanderfolgenden Windungen der in das Polymerrohr (30) eingebetteten spiralförmigen Spule in einem ersten Abschnitt des Polymerrohrs (30) eine erste konstante Steigung und in einem zweiten Abschnitt des Polymerrohrs (30) eine zweite konstante Steigung aufweisen, wobei die zweite konstante Steigung von der ersten konstanten Steigung verschieden ist.

9. Stent (10) nach einem der Ansprüche 1 bis 8, wobei der mindestens eine expandierbare Teil (22) ein Paar von Windungen in entgegengesetzten Richtungen umfasst, die U-förmig geschlossen sind, sodass der mindestens eine expandierbare Teil (22) in seiner expandierten Konfiguration einen Zylinder definiert.

10. Stent (10) nach Anspruch 9, ferner umfassend einen zweiten expandierbaren Teil (22), der ein Paar von Windungen in entgegengesetzten Richtungen umfasst, die U-förmig geschlossen sind, sodass der zweite expandierbare Teil (22) in seiner expandierten Konfiguration einen Zylinder definiert.

11. Stent (10) nach einem der Ansprüche 1 bis 8, wobei der mindestens eine expandierbare Teil (22) eine Reihe aufeinanderfolgender Windungen in der komprimierten Konfiguration umfasst und in seiner expandierten Konfiguration eine konische Form definiert.

12. Stent (10) nach einem der Ansprüche 1 bis 11, wobei der mindestens eine expandierbare Teil (22) mit einem Polymer beschichtet ist.

13. Stent (10) nach einem der Ansprüche 1 bis 12, wobei das Formgedächtnismaterial aus der Gruppe der Polymere oder Metalllegierungen ausgewählt ist, wobei das Formgedächtnismaterial vorzugsweise eine Metalllegierung aus Nickel und Titan ist.

14. Kit, umfassend:
• einen Stent (10) nach einem der Ansprüche 1 bis 13;
• ein Einführwerkzeug zum Einführen, Entfalten und Handhaben des Stents (10); und
• einen Spülkanal durch das Einführwerkzeug, um Flüssigkeit zum Stent (10) zu leiten.

## Revendications

1. Stent (10) destiné à être inséré dans la lumière d'un vaisseau ou d'un canal urologique, comprenant :
• un tube en polymère (30) dont la partie creuse définit un axe longitudinal (Oz) ;
• un fil unique (20) formant une bobine hélicoïdale comprenant :
i. une série de spires consécutives intégrées dans le tube en polymère (30) ; et
ii. au moins une partie extensible (22) pouvant se trouver soit dans une configuration comprimée, soit dans une configuration étendue, ladite partie extensible étant un matériau à mémoire de forme ;
dans lequel l'axe de la bobine hélicoïdale est sensiblement parallèle à l'axe longitudinal (Oz) ; et
dans lequel le tube en polymère (30) est radialement rigide pour empêcher une réduction du diamètre des spires intégrées dans le tube en polymère (30) lors de la torsion du stent (10) autour de l'axe longitudinal (Oz).

2. Stent (10) selon la revendication 1, dans lequel ladite au moins une partie extensible (22) est située à l'extrémité distale du stent (10) ou à l'extrémité proximale du stent (10).

3. Stent (10) selon la revendication 1 ou 2, dans lequel l'au moins une partie extensible (22) fait saillie devant l'extrémité distale du tube en polymère (30) ou devant l'extrémité proximale du tube en polymère (30).

4. Stent (10) selon la revendication 3, dans lequel l'au moins une partie extensible (22) comprend une série de spires consécutives.

5. Stent (10) selon la revendication 1 ou 2, dans lequel l'au moins une partie extensible (22) comprend une série de spires consécutives enroulées sur le tube en polymère (30) dans la configuration comprimée.

6. Stent (10) selon l'une quelconque des revendications 1 à 5, dans lequel
• la surface intérieure du tube en polymère (30) est cylindrique, de préférence la surface intérieure du tube en polymère (30) est cylindrique et lisse ; et/ou
• la surface extérieure du tube en polymère (30) est partiellement ou totalement ondulée.

7. Stent (10) selon l'une quelconque des revendications 1 à 6, dans lequel les spires consécutives de la bobine hélicoïdale intégrée dans le tube en polymère (30) ont un pas constant.

8. Stent (10) selon l'une quelconque des revendications 1 à 6, dans lequel les spires consécutives de la bobine hélicoïdale intégrée dans le tube en polymère (30) ont un premier pas constant dans une première partie du tube en polymère (30) et ont un deuxième pas constant dans une deuxième partie du tube en polymère (30), le deuxième pas constant étant différent du premier pas constant.

9. Stent (10) selon l'une quelconque des revendications 1 à 8, dans lequel l'au moins une partie extensible (22) comprend une paire de spires dans des directions opposées et fermées en forme de U, de sorte que l'au moins une partie extensible (22) dans sa configuration étendue définit un cylindre.

10. Stent (10) selon la revendication 9, comprenant en outre une deuxième partie extensible (22) comprenant une paire de spires dans des directions opposées et fermées en forme de U, de sorte que la deuxième partie extensible (22) dans sa configuration étendue définit un cylindre.

11. Stent (10) selon l'une quelconque des revendications 1 à 8, dans lequel l'au moins une partie extensible (22) comprend une série de spires consécutives dans la configuration comprimée et définit une forme conique dans sa configuration étendue.

12. Stent (10) selon l'une quelconque des revendications 1 à 11, dans lequel l'au moins une partie extensible (22) est revêtue d'un polymère.

13. Stent (10) selon l'une quelconque des revendications 1 à 12, dans lequel le matériau à mémoire de forme est choisi parmi le groupe des polymères ou des alliages métalliques, de préférence le matériau à mémoire de forme est un alliage métallique de nickel et de titane.

14. Kit comprenant :
• un stent (10) selon l'une quelconque des revendications 1 à 13 ;
• un outil d'introduction pour introduire, déployer et manipuler le stent (10) ; et
• un canal d'irrigation à travers l'outil d'introduction pour faire circuler un liquide vers le stent (10).
